# FASCICULE DE BREVET EUROPEEN

(11) **EP 3 047 025 B1**
(45) Date de publication et mention de la délivrance du brevet: **03.04.2019**
(21) Numéro de dépôt: 14796166.8
(22) Date de dépôt: 12.09.2014
(51) Int. Cl.: C12N 15/86

(54) **PROCEDE DE PRODUCTION DE VIRUS ENVELOPPES**
VERFAHREN ZUR HERSTELLUNG VON UMHÜLLTEN VIREN
METHOD FOR PRODUCING ENVELOPED VIRUSES

(30) Priorité: 16.09.2013 FR 1358909
(43) Date de publication de la demande: 27.07.2016
(73) Titulaire: Genethon, 91000 Evry (FR)
(72) Inventeur: FENARD, David, F-91540 Mennecy (FR)
(74) Mandataire: Cabinet Becker et Associés
(86) Numéro de dépôt international: PCT/FR2014/052279
(87) Numéro de publication internationale: WO 2015/036713

(56) Documents cités:
- WO-A1-2013/001041
- WO-A1-2013/076309
- MCTAGGART SALLY ET AL: "Effects of culture parameters on the production of retroviral vectors by a human packaging cell line", BIOTECHNOLOGY PROGRESS, AMERICAN INSTITUTE OF CHEMICAL ENGINEERS, US, vol. 16, no. 5, septembre 2000 (2000-09), pages 859-865, XP002333787, ISSN: 8756-7938, DOI: 10.1021/BP000078J
- "Protocol: Lenti-Viral Transfection", , 2 novembre 2010 (2010-11-02), pages 1-2, XP055127370, Extrait de l'Internet: URL:http://research.jax.org/faculty/mills/ protocols/lentiviral_transfection.pdf [extrait le 2014-07-07]
- MORIZONO K ET AL: "Transient low pH treatment enhances infection of lentiviral vector pseudotypes with a targeting Sindbis envelope", VIROLOGY, ELSEVIER, AMSTERDAM, NL, vol. 355, no. 1, 10 novembre 2006 (2006-11-10), pages 71-81, XP024896557, ISSN: 0042-6822, DOI: 10.1016/J.VIROL.2006.07.015 [extrait le 2006-11-10]
- FUMIKO HIGASHIKAWA ET AL: "Kinetic Analyses of Stability of Simple and Complex Retroviral Vectors", VIROLOGY, vol. 280, no. 1, février 2001 (2001-02), pages 124-131, XP055127252, ISSN: 0042-6822, DOI: 10.1006/viro.2000.0743 cité dans la demande
- NATHALIE HOLIC ET AL: "Influence of Mildly Acidic pH Conditions on the Production of Lentiviral and Retroviral Vectors", HUMAN GENE THERAPY CLINICAL DEVELOPMENT, vol. 25, no. 3, 1 septembre 2014 (2014-09-01), pages 178-185, XP055157649, ISSN: 2324-8637, DOI: 10.1089/humc.2014.027
- MORITA T ET AL: "Clastogenicity of low pH to various cultured mammalian cells", MUTATION RESEARCH, ELSEVIER, AMSTERDAM, NL, vol. 268, no. 2, 1 August 1992 (1992-08-01), pages 297-305, XP025206882, ISSN: 0027-5107, DOI: 10.1016/0027-5107(92)90235-T [retrieved on 1992-08-01]

## Description

L'invention concerne un procédé de production de virus enveloppés produits par culture cellulaire en milieu modérément acide. Les procédés de l'invention sont utiles pour la production de ces particules pour des applications en recherche biomédicale ou biotechnologique afin de faciliter les rendements de production notamment lorsqu'elle est réalisée à grande échelle et dans des conditions respectant les bonnes pratiques de fabrication (BPF).

### ARRIERE PLAN TECHNOLOGIQUE

Les vecteurs viraux enveloppés, et notamment les vecteurs lentiviraux tels que ceux dérivés du virus de l'immunodéficience humaine-1 (VIH-1) sont des outils prometteurs dans le cadre des approches de thérapie génique. Cependant, la production de masse de grade clinique de tels vecteurs reste un challenge à l'heure actuelle. Plusieurs approches ont été proposées pour améliorer leur production: optimisation de la transfection des plasmides nécessaires à la production du vecteur dans les cellules hôtes (e.g. optimisation de l'agent de transfection, de la densité cellulaire, du ratio de plasmides, etc.) ou des conditions de culture cellulaire focalisées sur des voies métaboliques cellulaires particulières (e.g. ajout de lipides, cholestérol, chloroquine, butyrate de sodium, etc.) (Ansorge et al. 2010; Schweizer and Merten 2010).

McTaggard et al., Biotechnology Progress, Vol. 16, no. 5, Septembre 2000, p. 859-865 décrit une étude des effets de paramètres de culture sur la production de vecteurs rétroviraux par des cellules d'empaquetage humaines.

Un document intitulé "Protocol: Lenti-Viral transfection/Transduction" (extrait de l'internet sur le site http://research.jax.org/faculty/mills/protocols/lentiviral_transfection.pdf) décrit un protocole pour la production de lentivirus.

Morizono et al., Virology, vol. 355, no. 1, 10 novembre 2006, décrit l'augmentation de l'infection de vecteurs lentiviraux pseudotypés au moyen d'une enveloppe de ciblage Sindbis lors d'un traitement transitoire à pH bas.

Les inventeurs ont eu l'idée d'élargir ce champ et d'optimiser des paramètres physico-chimiques, et se sont plus particulièrement intéressés aux conditions de pH. La neutralité du pH du milieu est considérée comme un paramètre critique pour la culture de cellules de mammifères. Par ailleurs, une étude a rapporté que des lentivirus pseudotypés avec les glycoprotéines d'enveloppe du virus de la stomatite vésiculeuse (VSV-G) sont instables à pH 6 en tampon phosphate (Higashikawa and Chang 2001). Au regard de ces éléments, l'homme du métier aurait considéré que la diminution du pH dans les milieux de culture aurait un impact négatif sur la production de virus enveloppés.

### RESUME DE L'INVENTION

La présente invention découle de l'observation de l'amélioration de la production de virus enveloppés lorsque les cellules productrices desdits virus sont cultivées en milieu modérément acide. De manière tout à fait surprenante, lesdites conditions modérément acides ont permis la production de lentivirus présentant des titres infectieux deux à trois fois supérieurs à ceux obtenus en milieu neutre classiquement utilisé.

L'invention a donc pour objet l'utilisation de conditions modérément acides pour la production d'un lentivirus. Plus particulièrement, l'invention concerne un procédé de production d'un vecteur lentiviral enveloppé, caractérisé en ce que le milieu de culture utilisé pour la culture des cellules hôtes productrices dudit vecteur est un milieu modérément acide de pH compris entre 5,8 et 6,2, le pH étant plus particulièrement de 6.

### DESCRIPTION DETAILLEE DE L'INVENTION

L'invention concerne donc un procédé pour la production d'un lentivirus caractérisé en ce que lesdits vecteurs sont produits en condition modérément acide.

L'expression "condition modérément acide" désigne le pH d'une solution aqueuse compris entre 5,8 et 6,2. Le pH sera notamment égal à 5,8, 5,9, 6, 6,1 ou 6,2. Selon un mode particulier de réalisation, le pH est d'environ 6. Le pH choisi dépendra également du pouvoir tampon du milieu utilisé, ce que l'homme du métier pourra déterminer facilement compte tenu de ses connaissances générales.

L'homme du métier est en mesure de modifier le pH d'une solution, notamment le pH d'un milieu de culture cellulaire. Il peut notamment introduire dans ladite solution une solution d'un acide, notamment d'un acide fort tel que l'acide chlorhydrique. Au besoin, une solution d'une base, notamment d'une base forte telle que l'hydroxyde de sodium, peut être utilisée pour réajuster le pH à la hausse pour atteindre la valeur souhaitée.

En dehors des conditions modérément acides qui font l'objet de la présente invention, le procédé employé de production de lentivirus met en oeuvre des procédés et matériaux bien connus dans le domaine. L'homme du métier peut se référer à ses connaissances générales dans la production de virus enveloppés, notamment représentées par (Ansorge et al. 2010; Schweizer and Merten 2010; Rodrigues et al. 2011).

Dans le cadre de la présente demande, le terme "virus" désigne aussi bien un virus naturel, tel que trouvé dans la nature, qu'un virus modifié, dont le génome comporte des modifications par rapport à celui du virus parental dont il est issu. Il peut s'agir d'un virus atténué, qui a perdu tout ou partie de son pouvoir pathogène par rapport au virus naturel dont il dérive. Son génome est modifié *in vivo* au cours des passages successifs en culture cellulaire ou dans un organisme vivant. Le terme "virus" peut faire également référence à un virus recombinant, dont le génome est modifié *in vitro* par les techniques du génie génétique. La modification peut, par exemple, permettre d'inactiver au moins un gène essentiel à la réplication virale (rendant le virus défectif pour la réplication) et/ou d'insérer un fragment d'ADN codant une protéine ou un ARN hétérologue (normalement non codée par le virus naturel). Dans ce dernier cas, on fait référence à un "vecteur viral". L'insertion a lieu dans une région appropriée du génome viral, de manière à permettre l'expression du fragment d'ADN hétérologue dans une cellule cible. Le terme "virus" désigne également les particules pseudo-virales, c'est-à-dire des particules virales soit sans glycoprotéine d'enveloppe à leur surface, soit sans génome et obtenues par l'assemblage spontané de protéines structurales et/ou enzymatiques du virus.

Selon un mode particulier de réalisation, le virus enveloppé est un vecteur viral. Le vecteur viral est notamment dérivé d'un rétrovirus, en particulier un lentivirus. Les vecteurs rétroviraux produits selon la présente divulgation sont notamment dérivés d'alpharétrovirus (tel que le VLA ou ALV en anglais pour avian leukosis virus), de bétarétrovirus (tel que le VTMM ou MMTV en anglais pour mouse mammary tumor virus), de gammarétrovirus (tel que les différents types de VLM ou MLV en anglais pour murine leukemia virus), de deltarétrovirus (tel que les différents types de VLTH ou HTLV en anglais pour human T-lymphotropic virus), d'epsilonrétrovirus (tel que le VSDW ou WDSV en anglais pour Walleye dermal sarcoma virus), de spumavirus (tel que le VFH ou HFV en anglais pour human foamy virus et le VFS ou SFV en anglais pour simian foamy virus), de lentivirus de primate tels que les différents types de virus de l'immunodéficience humaine (VIH ou HIV en anglais pour human immunodeficiency virus), les différents types de virus de l'immunodéficience simienne (VIS, ou SIV en anglais pour simian immunodeficiency virus), ou de lentivirus de mammifères non primates tels que le virus de l'anémie infectieuse équine (VAIE, ou EIAV en anglais pour equine infectious anemia virus), le virus de l'immunodéficience féline (VIF, ou FIV en anglais pour feline immunodeficiency virus), le virus de l'arthrite-encéphalite caprine (VAEC, ou CAEV en anglais pour caprine arthritis-encephalitis virus), ou le virus ovin Visna-maëdi (VVM ou VMV en anglais pour visna maedi virus).

Selon un mode particulier de réalisation, le vecteur rétroviral, en particulier lentiviral, est pseudotypé, c'est-à-dire qu'il comprend une glycoprotéine d'enveloppe dérivée d'un virus différent du virus dont est dérivée la particule rétrovirale, une glycoprotéine d'enveloppe modifiée ou une glycoprotéine d'enveloppe chimérique. Selon un mode particulier de réalisation, le vecteur rétroviral est pseudotypé avec une glycoprotéine d'enveloppe dérivée du virus de la stomatite vésiculeuse (VSV-G) ou du virus leucémogène du gibbon (GALV en anglais pour gibbon ape leukemia virus), bien que l'homme du métier puisse envisager l'utilisation d'autres glycoprotéines d'enveloppe virale (Frecha et al. 2008). Selon un mode particulier de réalisation, le vecteur rétroviral, plus particulièrement lentiviral, est pseudotypé avec une glycoprotéine d'enveloppe modifiée tel que GALVTR (glycoprotéine d'enveloppe de GALV dont l'extrémité C-terminale intravirion a été remplacée par l'extrémité C-terminale de la glycoprotéine d'enveloppe du virus leucémogène humain amphotrope A-MLV, permettant ainsi une incorporation de la glycoprotéine d'enveloppe très efficace dans la particule lentivirale) (Christodoulopoulos and Cannon 2001). Selon un mode particulier de réalisation, le vecteur rétroviral, plus particulièrement lentiviral, est pseudotypé avec une glycoprotéine d'enveloppe chimérique tel que la glycoprotéine d'enveloppe du virus de la rougeole dans laquelle a été insérée une protéine de fusion codant la région variable des chaînes lourde et légère d'une immunoglobuline (scFv en anglais pour single chain variable fragment) ou une protéine à domaines ankyrin répétés (DARPins en anglais pour designed ankyrin repeat proteins) pour permette le ciblage spécifique d'un récepteur donné à la surface des cellules cibles (Anliker et al. 2010; Munch et al. 2011).

Selon un mode particulier de réalisation, la glycoprotéine d'enveloppe virale utilisée pour pseudotyper le vecteur retroviral, plus particulièrement lentiviral, est dérivée d'une glycoprotéine d'enveloppe d'un virus appartenant à la famille des rhabdoviridae, notamment au genre vesiculovirus (e.g. VSV-G) ou au genre Lyssavirus (e.g. virus de la rage, virus Mokola); à la famille des Arenaviridae (e.g. le virus de la chorioméningite lymphocytaire (LCMV)); à la famille des togaviridae, plus particulièrement au genre alphavirus (e.g. virus de la Ross River (RRV), virus Sindbis, virus de la forêt de Semliki (SFV), virus de l'encéphalite équine vénézuelienne, virus de l'encéphalite équine de l'Ouest); à la famille des filoviridae, plus particulièrement au genre filovirus (e.g. virus Ebola, virus Lassa); à la famille des retroviridae, plus particulièrement au genre alpharétrovirus (e.g. virus de la leucose aviaire (ALV), virus du sarcome de Rous (RSV)), au genre bétarétrovirus (e.g. rétrovirus du mouton Jaagsiekte), au genre gammarétrovirus (e.g. différents virus de la leucémie murine (MLV), virus endogène du babouin sauvage (BAEV) ou modifié (BAEVTR), virus de la leucémie du Gibbon sauvage (GALV) ou modifié (GALVTR)), au genre deltaretrovirus (e.g. virus T-lymphtrophique humain (HTLV-1), au genre spumavirus (e.g. virus spumeux humain), au genre lentivirus (e.g. virus maedi-visna (MMV)); à la famille coronaviridae, plus particulièrement au genre coronavirus (e.g. SaRS-CoV); à la famille paramyxoviridae, plus particulièrement au genre respirovirus (e.g. virus de Sendai, virus parainfluenza de type 3 humain), au genre henipavirus (e.g. virus Nipah), au genre morbillivirus (e.g. virus de la rougeole); à la famille flaviridae, plus particulièrement au genre hepacivirus (e.g. virus de l'hépatite C (HCV)); à la famille orthomyxoviridae, plus particulièrement au genre influenza virus A (e.g. virus influenza); à la famille baculoviridae, plus particulièrement au genre nucleopolyhedrovirus (e.g. virus de la polyédrose nucléaire multiple d'Autographa californica). La glycoprotéine d'enveloppe utilisée pour le pseudotypage est plus particulièrement une glycoprotéine d'enveloppe modifiée, par exemple une protéine d'enveloppe fusionnée à un fragment d'anticorps à chaîne unique variable ScFV, telle qu'une glycoprotéine d'enveloppe rougeole-ScFV, Tupaia-ScFV, Sindbis-ScFV; une protéine d'enveloppe fusionnée à des domaines Ankirine répétés telle qu'une protéine d'enveloppe rougeole/DARPins (Measles/DARPins); ou encore une protéine d'enveloppe VSV-G + nanobody display à liaison défectueuse (binding-defective).

Selon un mode particulier de réalisation, le rétrovirus, plus particulièrement le lentivirus, produit est pseudotypé avec une glycoprotéine VSV-G, rougeole, GALV ou BAEV (si le virus est un rétrovirus), GALVTR ou BAEVTR (si le virus est un lentivirus) ou gp64 de baculovirus.

Le virus enveloppé peut par ailleurs contenir un transgène d'intérêt introduit dans son génome. Bien entendu, le transgène d'intérêt dépendra de l'utilisation spécifique pour laquelle le vecteur viral enveloppé est destiné. De manière illustrative, citons un transgène d'intérêt codant un ARN thérapeutique (e.g. un transgène d'intérêt codant un ARN antisens complémentaire d'une séquence cible ARN ou ADN), un transgène de thérapie génique codant une protéine déficiente ou absente chez un sujet atteint d'une pathologie, ou un transgène utilisé pour une vaccination par ADN, c'est-à-dire un transgène codant une protéine dont l'expression induira une vaccination de l'organisme receveur contre ladite protéine. Le procédé selon l'invention permet donc de produire un vecteur viral enveloppé utilisable en thérapie génique. Le procédé selon l'invention est avantageusement compatible avec les bonnes pratiques de laboratoires et permet d'envisager une production à grande échelle de vecteurs viraux enveloppés, notamment de vecteurs lentiviraux, en particulier de vecteurs lentiviraux pseudotypés (en particulier avec les protéines d'enveloppes VSV-G ou GALVTR).

Selon un mode préféré de réalisation pour la production d'un vecteur lentiviral, les quatre éléments suivants sont introduits dans la cellule hôte: une cassette d'expression comprenant un gène *gagpol* lentiviral, une cassette d'expression comprenant un gène rev lentiviral, une cassette d'expression d'un transgène d'intérêt, compris entre un LTR-5' et un LTR-3' lentiviral, et une cassette d'expression de glycoprotéine(s) d'enveloppe.

Dans un mode particulier de réalisation, le virus enveloppé, notamment un vecteur rétroviral, plus particulièrement un vecteur lentiviral, est produit à partir d'une lignée stable exprimant un ou plusieurs éléments nécessaires à la production d'un virus enveloppé (Miller 2001; Rodrigues et al. 2011), comme par exemple la lignée productrice humaine GPRG-EF1α-hχ_{c}OPT qui produit constitutivement un vecteur lentiviral dérivé du VIH-1 pseudotypé avec la glycoprotéine d'enveloppe VSV-G (Greene et al. 2012), ou par exemple la lignée productrice murine PG13-MFG-GFP qui produit constitutivement le vecteur gammarétroviral MLV pseudotypé avec la glycoprotéine d'enveloppe GALV (Merten 2004). Dans un mode particulier de réalisation, le virus enveloppé est produit à partir d'une cellule hôte de mammifère transfectée de manière transitoire avec un ou plusieurs plasmides codant les éléments nécessaires à la production du virus. Selon une variante permettant la production d'un vecteur lentiviral, lesdits éléments sont introduits dans la cellule au moyen de 4 plasmides: un plasmide portant une cassette d'expression comprenant un gène *gagpol* lentiviral, un plasmide portant une cassette d'expression comprenant un gène rev lentiviral, un plasmide de transfert comprenant une cassette d'expression d'un transgène d'intérêt, compris entre un LTR-5' et un LTR-3' lentiviral et un plasmide portant une cassette d'expression de glycoprotéine(s) d'enveloppe.

L'homme du métier comprendra de la présente divulgation que la mise en culture dans un milieu modérément acide est réalisée selon l'invention dès la mise en production du virus. C'est à dire que la cellule productrice est mise en culture dans un milieu dont le pH est modérément acide avant contact avec les cellules productrices. Selon un mode particulier de réalisation, la cellule est mise en culture dans un milieu modérément acide 5 à 24 heures après transfection, plus particulièrement 10 à 20 heures post-transfection, encore plus particulièrement 16 à 20 heures post-transfection.

La cellule hôte peut être choisie parmi toute cellule permettant la production d'un virus enveloppé. Selon un mode particulier de réalisation, ladite cellule est choisie parmi une cellule humaine (HEK293, HEK293T, HEK293FT, Te671, HT1080, CEM), une cellule de muridé (NIH-3T3), une cellule de mustélidé (Mpf), une cellule de canidé (D17) (Miller 2001; Miller et Chen 1996; Merten 2004; Rodrigues et al. 2011; Stacey et Merten, 2011).

Les cellules sont cultivées dans un milieu approprié pour la culture de cellules mammifères et la production d'un virus enveloppé. Le milieu peut par ailleurs être complémenté avec des additifs bien connus dans le domaine tels que des antibiotiques, du sérum (notamment du sérum de veau foetal, etc.) ajoutés dans des concentrations appropriées. Le milieu utilisé peut notamment comprendre du sérum ou en être dépourvu. Les milieux de culture de cellules mammifères sont bien connus dans le domaine. On peut citer à ce titre le milieu DMEM (Dulbecco's Modified Eagle's medium), le RPMI1640 ou un mélange de différents milieux de culture, incluant, par exemple, le DMEM/F12, ou un milieu sans sérum comme l'optiMEM®, l'optiPRO®, l'optiPRO-SFM®, le CD293®, le Freestyle F17® (Life Technologies) ou l'Ex-Cell® 293 (Sigma-Aldrich).

Dans les procédés employant des cellules transfectées de manière transitoire, tout agent permettant la transfection de plasmides peut être utilisé. Il peut être notamment fait emploi de phosphate de calcium ou de polyéthylèneimine, bien que d'autres agents puissent être envisagés par l'homme du métier (Ansorge et al. 2010). Les conditions (notamment quantité de plasmide(s), ratio entre les plasmides, ratio entre le(s) plasmide(s) et l'agent de transfection, le type de milieu, etc.) et la durée de transfection pourront être adaptées par l'homme du métier en fonction des caractéristiques du virus produit et/ou du transgène introduit dans le plasmide de transfert.

Le virus enveloppé est ensuite récolté à partir du surnageant de culture selon des méthodes bien connues dans le domaine.

Selon un mode particulier de réalisation, le procédé selon l'invention comprend les étapes suivantes:
- la transfection transitoire de cellules HEK293T au moyen d'un ou plusieurs plasmides codant les éléments nécessaires à la production dudit vecteur enveloppé;
- la culture desdites cellules dans un milieu approprié, dont le pH est d'environ 6;
- la récolte du virus enveloppé dans le surnageant de culture.
Selon une variante de ce mode de réalisation, le virus enveloppé produit est un lentivirus produit après transfection des cellules au moyen de quatre plasmides: un plasmide portant une cassette d'expression comprenant un gène *gagpol* lentiviral, un plasmide portant une cassette d'expression comprenant un gène rev lentiviral, un plasmide de transfert comprenant une cassette d'expression d'un transgène d'intérêt, compris entre un LTR-5' et un LTR-3' lentiviral et un plasmide portant une cassette d'expression de glycoprotéine(s) d'enveloppe. Selon une variante, la protéine d'enveloppe est dérivée du virus VSV (en particulier enveloppe VSV-G) ou du virus GALV (en particulier la glycoprotéine modifiée GALVTR pour les vecteurs lentiviraux).

Les virus ou vecteurs viraux produits peuvent ensuite être purifiés selon des procédés bien connus de l'homme de l'art (Segura et al. 2011).

Est également décrit un milieu de culture de cellules mammifères, ledit milieu étant modérément acide. En particulier, le milieu de culture est à un pH compris entre 5,5 et 6,6, plus particulièrement entre 5,8 et 6,2. Plus particulièrement, le pH du milieu de culture est d'environ 6. Selon un autre mode particulier de réalisation, le milieu de culture est DMEM modérément acide, notamment de pH tel que défini ci-avant. En particulier, le milieu de culture est un milieu DMEM de pH compris entre 5,8 et 6,2, en particulier un milieu DMEM de pH 6. Il est entendu que le milieu de culture est caractérisé par un pH modérément acide avant mise en culture des cellules.

L'invention concerne par ailleurs une trousse (ou kit) pour la mise en oeuvre du procédé de production de virus enveloppés tels que défini ci-dessus, comprenant un milieu de culture modérément acide, ou un milieu de culture accompagné d'une ou plusieurs solution(s) utiles pour amener le pH dudit milieu à une valeur modérément acide, la trousse comprenant en outre:
(a) un ou plusieurs plasmides appropriés pour la production du lentivirus, éventuellement pseudotypé; et
(b) des cellules appropriées pour la production dudit virus.

La trousse de l'invention est destinée à la production d'un lentivirus selon l'invention. Ainsi, elle peut en outre comprendre des instructions d'utilisation des différents constituants de la trousse permettant de produire un lentivirus selon l'invention. En particulier, ces instructions peuvent indiquer la manière dont les cellules destinées à la production doivent être transfectées avec les plasmides appropriés et cultivées dans le milieu de culture. En particulier, les instructions indiquent que les cellules productrices du lentivirus doivent être cultivées dans un milieu de culture de pH modérément acide tel que détaillé ci-dessus.

La présente demande concerne également une trousse pour la mise en oeuvre du procédé de production de virus enveloppés tels que défini ci-dessus, comprenant (i) des moyens pour la mise en oeuvre dudit procédé et (ii) des instructions à suivre pour la mise en oeuvre du procédé. Selon un mode particulier de réalisation, les moyens compris dans la trousse sont choisis parmi un ou plusieurs des moyens suivants:
(a) un ou plusieurs plasmides appropriés pour la production du virus enveloppé;
(b) des cellules appropriées pour la production dudit virus; et
(c) un milieu de culture modérément acide, ou un milieu de culture accompagné d'une ou plusieurs solution(s) utiles pour amener le pH dudit milieu à une valeur modérément acide. Une trousse selon l'invention peut ainsi notamment comprendre les moyens (a) et (b), (a) et (c), (b) et (c) ou (a) et (b) et (c).

La présente demande concerne également une trousse pour la mise en oeuvre du procédé selon de production de virus enveloppés tel que détaillé ci-dessus, comprenant un milieu de culture accompagné d'une ou plusieurs solution(s) utiles pour amener le pH dudit milieu à une valeur modérément acide.

### LEGENDE DES FIGURES

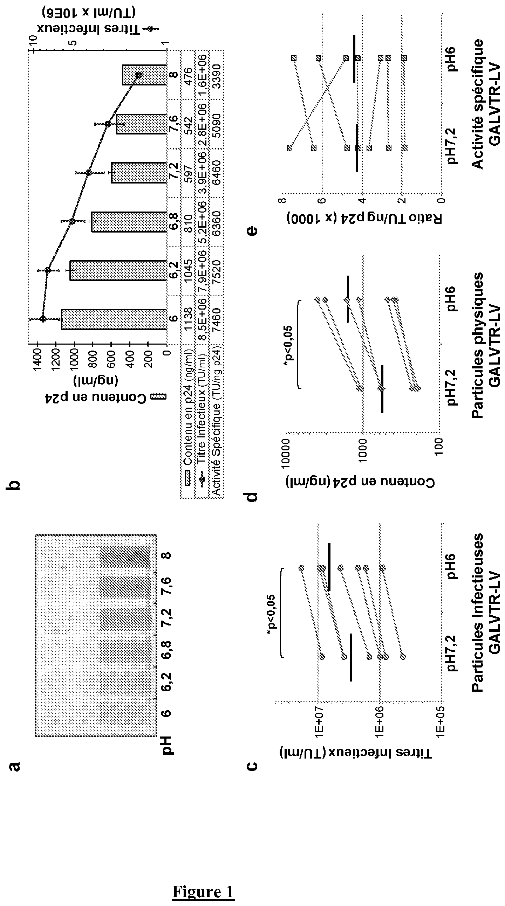

**Figure 1****.** Production d'un vecteur lentiviral (LV) pseudotypé avec l'enveloppe GALVTR (GALVTR-LV) dans diverses conditions de pH. **(a)** Les milieux de culture (DMEM/SVF) ont été tamponnés au pH indiqué à l'aide d'acide chlorhydrique ou d'hydroxyde de sodium. L'indicateur de pH contenu dans le milieu (rouge de phénol) présente une couleur allant du jaune (pH 6) au violet (pH 8). **(b)** des particules GALVTR-LV ont été produites à partir de cellules HEK293T cultivées en milieu DMEM/SVF à la valeur de pH indiqué. Les titres infectieux (TU/ml) ont été déterminés après transduction de cellules HCT116 et quantification du niveau d'expression du transgène GFP par cytométrie en flux. Le contenu des surnageants en particules physiques GALVTR-LV a été quantifié par mesure quantitative de la capside p24 du VIH-1 au moyen d'un kit ELISA commercial. L'activité spécifique correspondant au rapport entre les titres infectieux et la quantité de particules physiques (TU/ng de p24) est représentée sous les histogrammes. Les résultats représentent la moyenne de deux expériences indépendantes ± l'écart-type. Sept lots de vecteur GALVTR-LV ont été produits dans du milieu à pH 7,2 ou pH 6 et ont été titrés pour leur contenu en particules infectieuses (c) ou en particules physiques **(d). (e)** L'activité spécifique de chaque surnageant GALVTR-LV est représentée. Les barres indiquent la valeur moyenne des distributions.

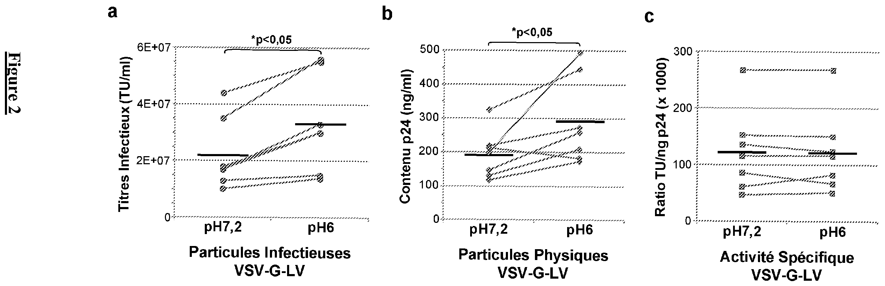

**Figure 2****.** Production d'un vecteur lentiviral VSV-G-LV dans des conditions de pH neutre ou légèrement acide. **(a)** Six lots de vecteur VSV-G-LV ont été produits à partir de cellules HEK293T cultivées dans du milieu DMEM/SVF au pH indiqué. Les titres infectieux ont été déterminés comme dans la fig. 1b. **(b)** La quantité de particules physiques a été déterminée par mesure quantitative de la capside p24 du VIH-1 au moyen d'un kit ELISA commercial. **(c)** L'activité spécifique de chaque surnageant VSV-G-LV est représentée. Les barres indiquent la valeur moyenne des distributions.

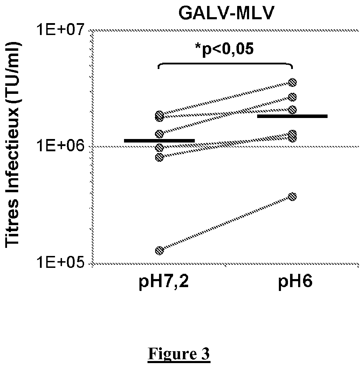

**Figure 3****.** Production d'un vecteur gammarétroviral GALV-MLV dans des conditions de pH neutre ou légèrement acide. Six lots de vecteur GALV-MLV ont été produits à partir de cellules HEK293T cultivées dans du milieu DMEM/SVF au pH indiqué. Les titres infectieux ont été déterminés comme dans la fig. 1b. Les barres indiquent la valeur moyenne des distributions.

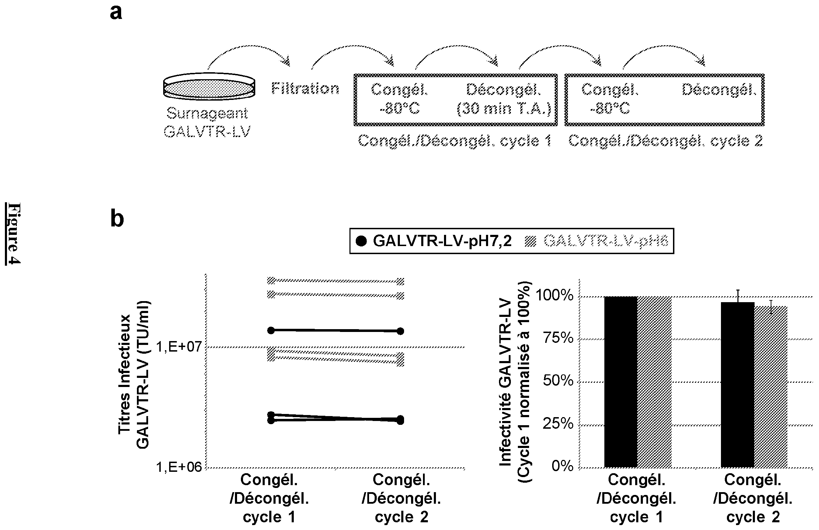

**Figure 4****.** Etude de la stabilité de particules lentivirales GALVTR-LV après plusieurs cycles de congélation/décongélation, **(a)** Représentation schématique du protocole de congélation/ décongélation des vecteurs. **(b)** Plusieurs lots de vecteur GALVTR-LV, produits à pH 7,2 (noir) ou pH 6 (gris), ont été exposés à un ou deux cycles de congélation/décongélation. Les titres infectieux ont été déterminés comme dans la fig. 1b. Les données représentent les différents titres infectieux obtenus (figure de gauche) ou ont été normalisées à 100% par rapport à la condition correspondant à un cycle de congélation/décongélation (figure de droite). Température Ambiante (T.A.), Congélation (Congél.), Décongélation (Décongél.).

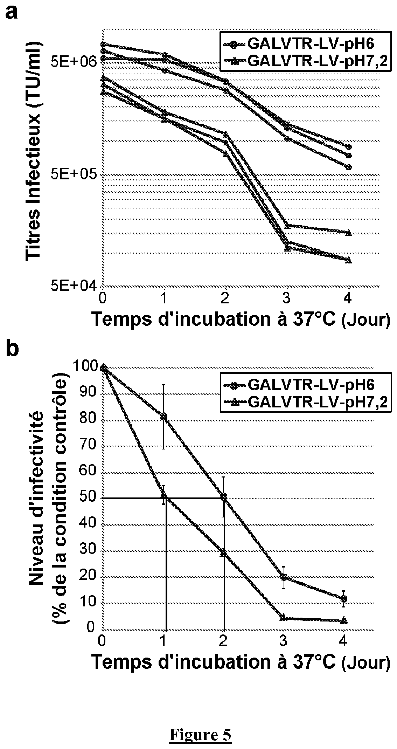

**Figure 5****.** Etude de la stabilité de particules lentivirales GALVTR-LV après exposition à une température de 37°C. Les cryotubes contenant un millilitre de particules GALVTR-LV, produits à pH 7,2 ou pH 6, ont été incubées à 37°C pendant 0 à 4 jours. Ensuite, les titres infectieux ont été déterminés comme dans la fig. 1b. **(a)** Les données sont représentées soit comme les titres infectieux obtenus à partir de trois expériences indépendantes ou **(b)** la moyenne des titres infectieux ± l'écart-type et normalisée à 100% par rapport à la condition contrôle (condition correspondant à un vecteur GALVTR-LV non exposé à 37°C).

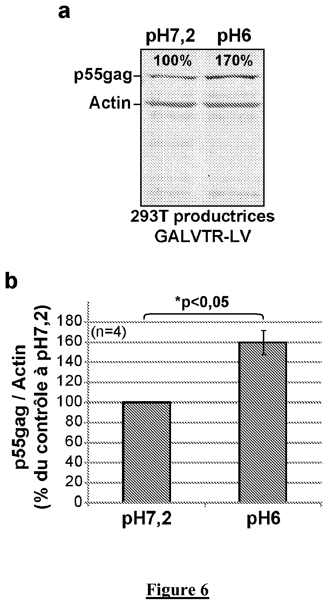

**Figure 6****.** Etude des niveaux d'expression de p55gag intracellulaire dans les cellules HEK293T productrices cultivées dans un milieu à pH neutre ou légèrement acide. **(a)** Western blot de l'expression de p55gag dans des lysats de cellules HEK293T produisant du vecteur GALVTR-LV à pH 7,2 ou pH 6. Le niveau d'expression de p55gag est normalisé par rapport au niveau d'expression de l'actine. **(b)** Les histogrammes représentent le niveau moyen d'expression de p55gag normalisé par rapport au niveau d'expression de l'actine dans quatre expériences indépendantes ± l'écart-type.

### EXEMPLES

### Matériel et méthodes

### Culture cellulaire

Des cellules HCT116 dérivées d'un carcinome colorectal humain (CCL-247; ATCC, Manassas, VA), des cellules HEK293T de rein embryonnaire humain (Merten et al. 2011), et des cellules productrices de gammarétrovirus GALV-MLV (lignée PG13-MFG-GFP) (Fenard et al. 2013) ont été cultivés à 37°C, 5 % de CO₂ dans du milieu Dulbecco's modified Eagle's medium (DMEM + Glutamax) supplémenté avec 2 à 10% de sérum de veau foetal (SVF) inactivé à la chaleur (Life Technologies, St-Aubin, France). Le milieu DMEM/SVF a été tamponné aux valeurs de pH indiquées en utilisant de l'acide chlorhydrique ou de l'hydroxyde de sodium, puis a été stérilisé sur filtre (0,22µ).

### Production de vecteur viraux et titration

Des vecteurs lentiviraux dérivés de VIH-1 ont été générés par transfection transitoire au phosphate de calcium de 4 plasmides dans des cellules HEK293T (Fenard et al. 2013) : les plasmides d'expression de gagpol (pKLgagpol) et rev (pBArev), le plasmide de transfert codant la protéine verte fluorescente GFP (pCCL-eGFP) et le plasmide codant la glycoprotéine d'enveloppe GALVTR (pBA.GALV/Ampho-Kana) ou VSV-G (pMDG). 16 à 20 h post-transfection, les cellules HEK293T ont été lavées et incubées dans du milieu DMEM/SVF tamponné à la valeur de pH indiquée, comprise entre 6 et 8. Après 24 h de production, les surnageants viraux ont été collectés, filtrés (0,45µ) et congelés à -80°C. Les titres de particules physiques ont été déterminés par mesure quantitative de la capside p24 du VIH-1 au moyen d'un kit ELISA commercial (Perkin Elmer, Courtaboeuf, France). Les titres infectieux ont été déterminés sur cellules HCT116 en détectant la GFP par cytométrie en flux (FACSCalibur, BD Biosciences, Le Pont de Claix, France), les titres étant exprimés en unités de transduction par millilitre (TU/ml) (Fenard et al. 2013).

### Exposition des vecteurs viraux à une température de 37°C et à de multiples cycles de congélation/décongélation

Des tubes de congelation de 1 ml contenant du surnageant GALVTR-LV (vecteur lentiviral pseudotypé avec la glycoprotéine d'enveloppe GALVTR) produit à pH 7,2 ou 6 ont été incubés pendant le temps indiqué à 37°C (les tubes à visse restant fermés). Puis, les tubes ont été congelés à nouveau à -80°C et des titrations sur cellules HCT116 ont été réalisées simultanément pour toutes les conditions afin de se prémunir des variations inter-expérimentales.

Pour les expériences de stabilité à la congélation/décongélation, les premier et second cycles de congélation/décongélation ont été réalisés en parallèle avec deux échantillons différents provenant de la même production de GALVTR-LV. Cette procédure a permis d'évaluer simultanément tous les titres infectieux GALVTR-LV afin d'éviter toute variabilité inter-expérimentale.

### Western blot et analyse

Les cellules productrices ont été lavées et lysées dans un tampon contenant 50 mM de Tris-HCl pH 7,5, 200 mM de NaCl, 1% de Triton X-100, 0,1% de SDS, 0,5% de désoxycholate de sodium, 10% de glycérol, 1 mM d'EDTA, 1mM de PMSF supplémenté d'un cocktail d'inhibiteurs de protéases (complete protease inhibitor cocktail, Roche Diagnostics, Meylan, France). Les concentrations en protéines ont été déterminées au moyen du kit Bio-Rad DC Protein Assay kit I (Bio-Rad, Marnes-la-Coquette, France). Les protéines (30 µg/piste) ont été séparées sur gel d'électrophorèse 10% SDS-polyacrylamide (PAGE), transférées sur membrance de nitrocellulose Hybond ECL (GE Healthcare Life Sciences, Velizy-Villacoublay, France) et un immunoblot a été réalisé en combinant un anticorps anti-p24 de chèvre (Abd Serotec, Oxford, UK) et un anticorps anti-actine de souris (clone AC-15) (Sigma-Aldrich, St-Quentin-Fallavier, France). Un anticorps d'âne anti-chèvre couplé à l'IRDye 800 et un anticorps d'âne anti-souris couplé à l'IRDye 680 ont été utilisés comme anticorps secondaires (Eurobio, Courtaboeuf, France). Les bandes immunoréactives ont été détectées avec le scanner Odyssey infrarouge et quantifiées avec le logiciel d'analyse Odyssey 3.0 (LI-COR Biosciences, Lincoln, NE).

### Analyses statistiques

Les valeurs P ont été déterminées avec le test non paramétrique de Wilcoxon au moyen du logiciel GraphPad Prism 5.

### Résultats

### Production d'un vecteur lentiviral GALVTR-LV dans un milieu de culture modérément acide

Les vecteurs lentiviraux (LV) pseudotypés au moyen de la glycoprotéine d'enveloppe GALVTR (GALVTR-LV) transduisent de manière très efficaces les cellules souches hématopoïétiques (Sandrin et al. 2002; Jacome et al. 2009). Cependant, la production à grande échelle de ce type de vecteurs reste un défi majeur. L'efficacité de production de vecteurs GALVTR-LV dans divers milieux de culture de pH 6 à 8 a été évaluée (figure 1a). La figure 1b montre que les titres infectieux obtenus à pH 8 sont fortement diminués par rapport au pH typique de 7,2. Au contraire, et de manière tout à fait surprenante, les titres infectieux de GALVTR-LV produits dans un milieu à pH 6 sont significativement supérieurs (2,3 x) à ceux obtenus à pH 7,2 (figure 1b et 1c). Il est important de noter que nous observons en parallèle une augmentation de la quantité d'antigène p24 dans les surnageants de GALVTR-LV produits à pH 6 (figures 1b et 1d). Cette corrélation positive conduit à une activité spécifique stable entre les vecteurs produits à pH neutre ou modérément acide (figure 1b et le), alors que cette activité spécifique diminue fortement à pH 8 (figure 1b). L'utilisation de conditions modérément acides représente donc la condition optimale pour produire de grandes quantités de vecteur GALVTR-LV.

### Effet des conditions du pH modérément acide sur la production de vecteurs lentiviraux pseudotypés avec la protéine VSV-G et sur des vecteurs gammarétroviraux MLV pseudotypés avec la protéine GALV

Les résultats encourageants obtenus avec le vecteur GALVTR-LV nous ont poussés à tester ces mêmes conditions pour la production d'un vecteur lentiviral pseudotypé avec une glycoprotéine d'enveloppe, utilisée très largement dans le domaine, la protéine VSV-G (vecteurs VSV-G-LV). La figure 3 montre qu'un milieu à pH 6 permet l'augmentation significative de la production de particules VSV-G-LV infectieuses (figure 2a) et physiques (figure 2b), en moyenne d'un facteur 1,5, avec une activité spécifique stable (figure 2c). Les effets délétères d'un pH égal à 6 rapportés par l'équipe d'Higashikawa (*op. cit*.) sont probablement une conséquence du protocole utilisé par ces auteurs, qui ont produits les particules rétrovirales à pH neutre, les ont concentrées puis diluées dans une solution non ionique tamponnée à pH 6, entraînant une perte d'infectivité du surnageant viral de 90 %. Contre toute attente, les particules VSV-G-LV, produites directement dans un milieu de culture à pH 6 supplémenté en SVF, sont non seulement stables mais également, contre toute attente, produite à un niveau plus élevé que lorsqu'elles sont produites à partir d'un milieu de culture à pH 7,2, classiquement reconnu comme optimal pour ce type de production.

Afin de s'assurer que l'amélioration observée n'est pas dépendante des cellules HEK293T utilisée, ou à la production des seuls vecteurs lentiviraux, l'effet du pH modérément acide a été évalué sur la lignée cellulaire PG13-MFG-GFP, productrice de GALV-MLV (gammarétrovirus MLV pseudotypé avec la glycoprotéine d'enveloppe GALV) (Merten 2004). La lignée cellulaire PG13 originale est une lignée cellulaire de fibroblastes murins (NIH-3T3) transfectée stablement avec un système de packaging du virus MLV (pLGPS) et une construction codant la glycoprotéine d'enveloppe GALV (pMOV-GALV) (Miller et al. 1991). Pour produire de manière constitutive les pseudotypes GALV-MLV rétroviraux, le plasmide de transfert codant la protéine GFP placée sous contrôle du promoteur LTR du MLV (pMFG-GFP) a été stablement introduit dans la lignée PG13. Dans des cultures cellulaires réalisées en parallèle, les cellules PG13-MFG-GFP ont été incubées dans du DMEM tamponné à pH 7,2 ou à pH 6 et 24 à 48 heures plus tard, le contenu en particules infectieuses dans les surnageants récoltés a été évalué. La figure 3 montre que la production de particules GALV-MLV est augmentée de manière significative à pH modérément acide. Ce résultat est particulièrement intéressant puisqu'il montre que le procédé de production proposé n'est pas limité à une lignée cellulaire humaine telle que la lignée HEK293T, et qu'en plus d'être particulièrement adapté à la production de vecteurs lentiviraux, il n'est pas limité à la production de vecteurs du genre lentivirus puisque d'autres virus enveloppés peuvent être produits de manière plus efficace au moyen du protocole de l'invention.

### Stabilité des particules GALVTR-LV exposées à plusieurs cycles de congélation / décongélation

Les surnageants de vecteurs lentiviraux récoltés sont généralement stockés à -80°C avant purification. On aurait pu supposer que les conditions de pH modérément acide auraient eu pour effet délétère d'augmenter l'inactivation des virions pendant la procédure de congélation ou de décongélation. Les surnageants de particules GALVTR-LV ont donc été soumis à un ou deux cycles de congélation / décongélation, les titres infectieux ayant été déterminés à chaque étape de décongélation (figure 4a). La figure 4b montre que les conditions modérément acides n'affectent pas l'infectivité des particules. La diminution moyenne en titres infectieux après deux cycles de congélation / décongélation par rapport à un seul cycle n'est que de 5 % aussi bien à pH 7,2 qu'à pH 6. L'infectivité n'est donc pas altérée lorsque les vecteurs lentiviraux sont congelés en conditions modérément acides.

### Effet de l'exposition à long terme des particules GALVTR-LV à une température de 37°C

Lors de la transduction lentivirale, les cellules cibles, qui sont dans notre cas des cellules de mammifères, sont cultivées à une température de 37°C. Nous avons donc cherché à savoir si la production de vecteurs lentiviraux à un pH acide doux avait un effet délétère sur leur stabilité après une exposition plus ou moins longue à une température de 37°C. Pour cela, les tubes de congélation contenant du surnageant de vecteurs GALVTR-LV produits à pH 7,2 ou à pH 6 ont été incubés pendant 0 à 4 jours à 37°C et la cinétique de diminution de l'infectivité a été suivie. Comme le montre la figure 5a, alors que les titres infectieux, après une longue exposition à 37°C, sont fortement réduits, aussi bien pour les vecteurs GALVTR-LV produits à pH 7,2 que pour les vecteurs produits à pH 6, la pente de cette diminution est moins prononcée pour les vecteurs GALVTR-LV produits à pH 6. La demi-vie qui en résulte pour les vecteurs GALVTR-LV produits à pH 6 est le double de celle observée pour les vecteurs GALVTR-LV produits à pH 7 (autour de 2 jours versus un jour, voir Figure 5b). De façon intéressante, cette expérience a montré que les surnageants bruts de vecteurs GALVTR-LV sont plutôt résistants (demi-vie de un à 2 jours) à une température de 37°C dans un environnement clos (tube à visse fermé). Ceci est à mettre en opposition avec la stabilité en culture cellulaire montrant une demi-vie de seulement 6 h (Strang et al. 2004), ce qui suggère que d'autres paramètres que la température, tels que le stress oxydatif, doivent aussi être pris en considération lorsque la stabilité des vecteurs lentiviraux est évaluée en culture cellulaire à 37°C.

### Modulation du niveau d'expression intracellulaire de p55gag dans les cellules productrices HEK293T cultivées à pH modérément acide.

La quantité de protéines p24 de VIH-1 récoltée dans les surnageants GALVTR-LV est améliorée en conditions modérément acides (figures 1b et 1d). Nous avons donc cherché à déterminer si cette augmentation pouvait être la conséquence d'une augmentation du niveau d'expression intracellulaire de la protéine précurseur p55gag de VIH-1 dans les cellules productrices. Dans la figure 6a, une expérience d'immunoblotting montre une augmentation de l'expression intracellulaire de p55gag à pH 6 par rapport au pH 7,2, avec une surexpression moyenne de 160% (figure 6b). Cette corrélation positive entre la surexpression de p55gag intracellulaire et l'augmentation des quantités de protéine p24 dans les surnageants lentiviraux suggère que les conditions modérément acides créent un environnement plus favorable à une expression optimale des composants viraux.

### REFERENCES BIBLIOGRAPHIQUES

Anliker, B., T. Abel, S. Kneissl, J. Hlavaty, A. Caputi, J. Brynza, et al. (2010). "Specific gene transfer to neurons, endothelial cells and hematopoietic progenitors with lentiviral vectors." Nat. Methods 7(11):929-935.
Ansorge, S., O. Henry and A. Kamen (2010). "Recent progress in lentiviral vector mass production." Biochem. Eng. J. 48(3): 362-377.
Christodoulopoulos, I. and P. M. Cannon (2001). "Sequences in the cytoplasmic tail of the gibbon ape leukemia virus envelope protein that prevent its incorporation into lentivirus vectors." J. Virol. 75(9): 4129-4138.
Fenard, D., D. Ingrao, A. Seye, J. Buisset, S. Genries, S. Martin, et al. (2013). "Vectofusin-1, a new viral entry enhancer, strongly promotes lentiviral transduction of human hematopoietic stem cells." Mol Ther Nucleic Acids 2: e90.
Frecha, C., J. Szecsi, F. L. Cosset and E. Verhoeyen (2008). "Strategies for targeting lentiviral vectors." Curr. Gene Ther. 8(6): 449-460.
Greene, M. R., T. Lockey, P. K. Mehta, Y. S. Kim, P. W. Eldridge, J. T. Gray, et al. (2012). "Transduction of human CD34+ repopulating cells with a self-inactivating lentiviral vector for SCID-X1 produced at clinical scale by a stable cell line." Hum Gene Ther Methods 23(5): 297-308.
Higashikawa, F. and L. Chang (2001). "Kinetic analyses of stability of simple and complex retroviral vectors." Virology 280(1): 124-131.
Jacome, A., S. Navarro, P. Rio, R. M. Yanez, A. Gonzalez-Murillo, M. L. Lozano, et al. (2009). "Lentiviral-mediated genetic correction of hematopoietic and mesenchymal progenitor cells from Fanconi anemia patients." Mol. Ther. 17(6): 1083-1092.
Merten, O. W. (2004). "State-of-the-art of the production of retroviral vectors." J. Gene Med. 6 Suppl 1: S105-124.
Merten, O. W., S. Charrier, N. Laroudie, S. Fauchille, C. Dugue, C. Jenny, et al. (2011). "Large-scale manufacture and characterization of a lentiviral vector produced for clinical ex vivo gene therapy application." Hum. Gene Ther. 22(3): 343-356.
Miller, A. D. (2001). "Production of retroviral vectors." Curr. Protoc. Hum. Genet. Chapter 12: Unit 12 15.
Miller, A. D., J. V. Garcia, N. von Suhr, C. M. Lynch, C. Wilson and M. V. Eiden (1991). "Construction and properties of retrovirus packaging cells based on gibbon ape leukemia virus." J Virol 65(5): 2220-2224.
Miller AD, Chen F. (1996) Retrovirus packaging cells based on 10A1 murine leukemia virus for production of vectors that use multiple receptors for cell entry. J. Virol. 70: 5564-5571.
Munch, R. C., M. D. Muhlebach, T. Schaser, S. Kneissl, C. Jost, A. Pluckthun, et al. (2011). "DARPins: an efficient targeting domain for lentiviral vectors." Mol. Ther. 19(4): 686-693.
Rodrigues, A. F., P. M. Alves and A. S. Coroadinha (2011). Production of Retroviral and Lentiviral Gene Therapy Vectors: Challenges in the Manufacturing of Lipid Enveloped Virus. Viral Gene Therapy. K. Xu, InTech. Chapter 2: 15-40.
Sandrin, V., B. Boson, P. Salmon, W. Gay, D. Negre, R. Le Grand, et al. (2002). "Lentiviral vectors pseudotyped with a modified RD114 envelope glycoprotein show increased stability in sera and augmented transduction of primary lymphocytes and CD34+ cells derived from human and nonhuman primates." Blood 100(3): 823-832.
Schweizer, M. and O. W. Merten (2010). "Large-scale production means for the manufacturing of lentiviral vectors." Curr. Gene Ther. 10(6): 474-486.
Segura, M. M., A. A. Kamen and A. Garnier (2011). "Overview of current scalable methods for purification of viral vectors." Methods Mol Biol 737: 89-116.
Stacey GN, Merten O-W (2011) Chapter 3: Hosts cells and cell banking. In: Merten O-W, Al-Rubeai M (eds.): Viral Vectors for Gene Therapy: Methods and Protocols, in the series of: Methods in Molecular Biology 737, Humana Press, New York, NY, pp 45-88.
Strang, B. L., Y. Ikeda, F. L. Cosset, M. K. Collins and Y. Takeuchi (2004). "Characterization of HIV-1 vectors with gammaretrovirus envelope glycoproteins produced from stable packaging cells." Gene Ther. 11(7): 591-598.

## Revendications

1. Procédé de production d'un lentivirus, éventuellement pseudotypé, **caractérisé en ce que** les cellules hôtes productrices dudit lentivirus sont mises en culture dans un milieu tamponné modérément acide, le pH du milieu étant compris entre 5,8 et 6,2, le pH étant plus particulièrement de 6.

2. Procédé selon la revendication 1, le lentivirus étant pseudotypé avec une glycoprotéine d'enveloppe dérivée d'un virus appartenant à une famille choisie parmi rhabdoviridae, arenaviridae, togaviridae, filoviridae, retroviridae, coronaviridae, paramyxoviridae, flaviridae, orthomyxoviridae ou baculoviridae.

3. Procédé selon la revendication 1 ou 2, le lentivirus étant pseudotypé avec une glycoprotéine d'enveloppe choisie parmi la glycoprotéine d'enveloppe dérivée du virus de la stomatite vésiculeuse (VSV-G), virus de la rage, virus Mokola, virus de la chorioméningite lymphocytaire (LCMV), virus de la Ross River (RRV), virus Sindbis, virus de la forêt de Semliki (SFV), virus de l'encéphalite équine vénézuelienne, virus de l'encéphalite équine de l'Ouest, virus Ebola, virus Lassa, virus de la leucose aviaire (ALV), virus du sarcome de Rous (RSV), rétrovirus du mouton Jaagsiekte, virus de la leucémie murine (MLV), virus endogène du babouin modifié (BAEVTR), virus de la leucémie du Gibbon sauvage modifié (GALVTR), virus T-lymphtrophique humain (HTLV-1), virus spumeux humain, virus maedi-visna (MMV), SaRS-CoV, virus de Sendai, virus parainfluenza de type 3 humain, virus Nipah, virus de la rougeole, virus de l'hépatite C (HCV), virus influenza, ou du virus de la polyédrose nucléaire multiple d'Autographa californica.

4. Procédé selon la revendication 3, le lentivirus étant pseudotypé avec une protéine d'enveloppe choisie parmi la protéine d'enveloppe VSV-G ou la protéine d'enveloppe GALVTR.

5. Procédé selon l'une quelconque des revendications précédentes, la cellule hôte étant choisie parmi une cellule HEK293, HEK293T, HEK293FT, Te671, CEM, NIH-3T3, Mpf ou D17.

6. Procédé selon l'une quelconque des revendications précédentes, la cellule hôte étant transfectée de manière stable ou de manière transitoire avec un ou plusieurs plasmides codant les éléments nécessaires à la production du lentivirus.

7. Procédé selon l'une quelconque des revendications précédentes, le lentivirus contenant en outre un transgène d'intérêt dans son génome.

8. Procédé selon l'une quelconque des revendications précédentes, ledit procédé comprenant les étapes suivantes:
- la transfection transitoire de cellules HEK293T au moyen d'un ou plusieurs plasmides codant les éléments nécessaire à la production dudit lentivirus, éventuellement pseudotypé;
- la culture desdites cellules dans un milieu approprié, dont le pH est de 6;
- la récolte du lentivirus, éventuellement pseudotypé, dans le surnageant de culture.

9. Procédé selon la revendication 8, les cellules étant transfectées au moyen de quatre plasmides: un plasmide portant une cassette d'expression comprenant un gène *gagpol* lentiviral, un plasmide portant une cassette d'expression comprenant un gène rev lentiviral, un plasmide de transfert comprenant une cassette d'expression d'un transgène d'intérêt, compris entre un LTR-5' et un LTR-3' lentiviral et un plasmide portant une cassette d'expression de glycoprotéine(s) d'enveloppe.

10. Trousse pour la mise en oeuvre du procédé selon l'une quelconque des revendications 1 à 9, comprenant soit (i) un milieu de culture tamponné modérément acide de pH compris entre 5,8 et 6,2, plus particulièrement de pH 6, soit (ii) un milieu de culture accompagné d'une ou plusieurs solution(s) utile(s) pour amener le pH dudit milieu à une valeur modérément acide comprise entre 5,8 et 6,2, le pH étant plus particulièrement de 6, la trousse comprenant en outre:
(a) un ou plusieurs plasmides appropriés pour la production du lentivirus, éventuellement pseudotypé; et
(b) des cellules appropriées pour la production dudit virus ladite trousse comprenant éventuellement des instructions pour la mise en oeuvre du procédé de production d'un lentivirus, éventuellement pseudotypé selon l'une quelconque des revendications 1 à 9.

## Patentansprüche

1. Verfahren zur Herstellung eines gegebenenfalls pseudotypisierten Lentivirus, **dadurch gekennzeichnet, dass** die besagtes Lentivirus produzierenden Wirtszellen in einem mäßig sauer gepufferten Medium kultiviert werden, wobei der pH des Mediums zwischen 5,8 und 6,2 beträgt, insbesondere der pH 6 beträgt.

2. Verfahren gemäß Anspruch 1, wobei das Lentivirus mit einem Hüllglycoprotein pseudotypisiert ist, das von einem Virus stammt, das zu einer aus Rhabdoviridae, Arenaviridae, Togaviridae, Filoviridae, Retroviridae, Coronaviridae, Paramyxoviridae, Flaviridae, Orthomyxoviridae oder Baculoviridae ausgewählten Familie gehört.

3. Verfahren gemäß Anspruch 1 oder 2, wobei das Lentivirus mit einem Hüllglycoprotein pseudotypisiert ist, ausgewählt aus dem Hüllglycoprotein, das vom Vesicular-Stomatitis-Virus (VSV-G), Tollwutvirus, Mokola-Virus, lymphocytären Choriomeningitis-Virus (LCMV), Ross-River-Virus (RRV), Sindbis-Virus, Semliki-Forest-Virus (SFV), Venezolanischen Pferdeenzephalitis-Virus, Westlichen Pferdeenzephalitis-Virus, Ebola-Virus, Lassa-Virus, Aviären Leukosevirus (ALV), Rous-Sarkom-Virus (RSV), Jaagsiekte-Schaf-Retrovirus, Murinen Leukämievirus (MLV), modifizierten Baboon endogenous virus (BaEVTR), modifizierten Gibbonaffen-Leukämievirus (GALVTR), humanen T-lymphotropen-Virus (HTLV-1), humanen Spumavirus, Maedi-Visna-Virus (MMV), SaRS-CoV, Sendai-Virus, humanen Parainfluenzavirus Typ 3, Nipah-Virus, Masernvirus, Hepatitis-C-Virus (HCV), Influenzavirus oder Autographa californica Nucleopolyhedrovirus stammt.

4. Verfahren gemäß Anspruch 3, wobei das Lentivirus mit einem Hüllprotein pseudotypisiert ist, ausgewählt aus dem VSV-G-Hüllprotein oder dem GALVTR-Hüllprotein.

5. Verfahren gemäß einem der vorhergehenden Ansprüche, wobei die Wirtszelle aus einer HEK293-, HEK293T-, HEK293FT-, Te671-, CEM-, NIH-3T3-, Mpf- oder D17-Zelle ausgewählt ist.

6. Verfahren gemäß einem der vorhergehenden Ansprüche, wobei die Wirtszelle mit einem oder mehreren Plasmiden stabil oder transient transfiziert ist, welche die für die Herstellung des Lentivirus erforderlichen Elemente codieren.

7. Verfahren gemäß einem der vorhergehenden Ansprüche, wobei das Lentivirus außerdem ein Transgen von Interesse in seinem Genom enthält.

8. Verfahren gemäß einem der vorhergehenden Ansprüche, wobei besagtes Verfahren folgende Schritte umfasst:
- die transiente Transfektion von HEK293T-Zellen mit einem oder mehreren Plasmiden, welche die für die Herstellung besagten gegebenenfalls pseudotypisierten Lentivirus erforderlichen Elemente codieren;
- Kultivierung besagter Zellen in einem geeigneten Medium, dessen pH 6 beträgt;
- Ernten des gegebenenfalls pseudotypisierten Lentivirus im Kulturüberstand.

9. Verfahren gemäß Anspruch 8, wobei die Zellen mit vier Plasmiden transfiziert sind: einem Plasmid, das eine Expressionskassette trägt, umfassend ein lentivirales *gagpol* Gen, einem Plasmid, das eine Expressionskassette trägt, umfassend ein lentivirales rev Gen, einem Transferplasmid, umfassend eine Expressionskassette eines Transgens von Interesse zwischen einem lentiviralen 5'-LTR und einem lentiviralen 3'-LTR, und einem Plasmid, das eine Expressionskassette des oder der Hüllglycoproteine trägt.

10. Kit zur Durchführung des Verfahrens gemäß einem der Ansprüche 1 bis 9, umfassend entweder (i) ein Kulturmedium, mäßig sauer gepuffert mit einem pH zwischen 5,8 und 6,2, insbesondere pH 6, oder (ii) ein Kulturmedium und eine oder mehrere Lösungen, die zum Einstellen des pH besagten Mediums auf einen mäßig sauren Wert zwischen 5,8 und 6,2 zweckdienlich sind, wobei der pH insbesondere 6 beträgt und das Kit außerdem umfasst:
(a) ein oder mehrere Plasmide, die für die Herstellung des gegebenenfalls pseudotypisierten Lentivirus geeignet sind, und
(b) Zellen, die für die Herstellung besagten Virus geeignet sind, wobei besagtes Kit gegebenenfalls Anleitungen für die Durchführung des Verfahrens zur Herstellung eines gegebenenfalls pseudotypisierten Lentivirus gemäß einem der Ansprüche 1 bis 9 umfasst.

## Claims

1. A process for producing a lentivirus, optionally pseudotyped, **characterized in that** the host cells producing said lentivirus are cultured in a mildly acidic buffered medium, the pH of the medium being comprised between 5.8 and 6.2, the pH being more particularly 6.

2. The process according to claim 1, the lentivirus being pseudotyped with an envelope glycoprotein derived from a virus belonging to a family selected from *Rhabdoviridae, Arenaviridae, Togaviridae, Filoviridae, Retroviridae, Coronaviridae, Paramyxoviridae, Flaviviridae, Orthomyxoviridae* or *Baculoviridae.*

3. The process according to claim 1 or 2, the lentivirus being pseudotyped with an envelope glycoprotein selected from envelope glycoprotein derived from vesicular stomatitis virus (VSV-G), rabies virus, Mokola virus, lymphocyte choriomeningitis virus (LCMV), Ross River virus (RRV), Sindbis virus, Semliki Forest virus (SFV), Venezuelan equine encephalitis virus, west equine encephalitis virus, Ebola virus, Lassa virus, avian leukosis virus (ALV), Rous sarcoma virus (RSV), jaagsiekte sheep retrovirus, murine leukaemia virus (MLV), modified baboon ape endogenous virus (BAEVTR), modified gibbon ape leukaemia virus (GALVTR), human T-lymphotropic virus (HTLV-1), human spumavirus, maedi-visna virus (MMV), SaRS-CoV, Sendai virus, human parainfluenza virus type 3, Nipah virus, measles virus, hepatitis C virus (HCV), influenza virus, or *Autographa californica* multiple nuclear polyhedrosis virus.

4. The process according to claim 3, the lentivirus being pseudotyped with an envelope protein selected from the VSV-G envelope protein or the GALVTR envelope protein.

5. The process according to any one of the preceding claims, the host cell being selected from a HEK293, HEK293T, HEK293FT, Te671, CEM, NIH-3T3, Mpf or D17 cell.

6. The process according to any one of the preceding claims, the host cell being stably or transiently transfected with one or more plasmids encoding the elements necessary for the production of the lentivirus.

7. The process according to any one of the preceding claims, the lentivirus further containing a transgene of interest in its genome.

8. The process according to any one of the preceding claims, said process comprising the following steps:
- transient transfection of HEK293T cells by means of one or more plasmids encoding the elements necessary for the production of said lentivirus, optionally pseudotyped;
- culturing said cells in an appropriate medium, the pH of which is 6;
- harvesting the lentivirus, optionally pseudotyped, in the culture supernatant.

9. The process according to claim 8, the cells being transfected by means of four plasmids: one plasmid carrying an expression cassette comprising a lentiviral *gagpol* gene, one plasmid carrying an expression cassette comprising a lentiviral rev gene, one transfer plasmid comprising an expression cassette for a transgene of interest, comprised between a lentiviral LTR-5' and LTR-3', and one plasmid carrying an envelope glycoprotein(s) expression cassette.

10. A kit for carrying out the process according to any one of claims 1 to 9, comprising either (i) a mildly acidic buffered culture medium of pH comprised between 5.8 and 6.2, more particularly of pH 6, or (ii) a culture medium accompanied by one or more solution(s) useful for bringing the pH of said medium to a mildly acidic value comprised between 5.8 and 6.2, the pH being more particularly 6, the kit further comprising:
(a) one or more plasmids suitable for producing the lentivirus, optionally pseudotyped; and
(b) cells suitable for producing said virus, said kit optionally including instructions for carrying out the process for producing a lentivirus, optionally pseudotyped, according to any one of claims 1 to 9.
